# EUROPEAN PATENT APPLICATION

(11) **EP 1 882 747 A1**
(43) Date of publication of application: **30.01.2008**
(21) Application number: 07112945.6
(22) Date of filing: 23.07.2007
(51) Int. Cl.: C12Q 1/68

(54) **A method for the analysis of the methylation status of a nucleic acid**

(30) Priority: 16.10.2006 EP 06122381; 27.07.2006 EP 06076487
(71) Applicant: Epigenomics AG, 10178 Berlin (DE)
(72) Inventor: Krispin, Manuel, 10827 Berlin (DE); Distler, Jürgen, Dr., 12163 Berlin (DE); Heiden, Esmeralda, Dr., 10589 Berlin (DE)

(57) **Abstract**

The invention relates to a method for the analysis of the methylation status of a nucleic acid. According to the invention, the method comprises following steps: (a) treating the nucleic acid with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases, (b) hybridizing to the treated nucleic acid an at least one oligonucleotide, (c) ligating the at least one oligonucleotide to itself to form a circular DNA molecule if a particular methylation status is present in the nucleic acid, and (d) amplifying the DNA molecule formed to detect the methylation status of the nucleic acid.

Sequence listing

<110> Epigenomics AG

<120> A method for the analysis of the methylation status of a nucleic acid

<160> 10

<210> 1
<211> 20
<212> DNA
<213> Homo Sapiens

<400> 1

atgutgaucg cgaguaguaa                                                    20

<210> 2
<211> 10
<212> DNA
<213> Homo Sapiens

<400> 2

cgatcaacat                                                               10

<210> 3
<211> 10
<212> DNA
<213> Homo Sapiens

<400> 3

ttactactcg                                                               10

<210> 4
<211> 125
<212> DNA
<213> Homo Sapiens

<400> 4

ctaaaaacca ttatataaac ttcccctacg gctcaacgtt cctattcggt tuuuttgcaa      60
atgttatcga ggtccggcac gcacaggtgg cgaatctctt taaatcaaaa accctacccc     120
aaaac                                                                 125

<210> 5
<211> 142
<212> DNA
<213> Homo Sapiens

<400> 5

ggagtaaata cagcaggcga aggggaagct cacacaatgg tctccagcgc tctggggcag      60
ggcttctgag gggcgggcct gcctctgccg ggacctggag cccccgcccc tcggagaggc     120
tcctaggctg acttgggcag ag                                              142

<210> 6
<211> 142
<212> DNA
<213> Homo Sapiens

<400> 6

ggagtaaata tagtaggcga aggggaagtt tatataatgg tttttagcgt tttggggtag      60
ggtttttgag gggcgggttt gtttttgtcg ggatttggag ttttcgtttt tcggagaggt     120
ttttaggttg atttgggtag ag                                              142

<210> 7

<211> 22
<212> DNA
<213> Homo Sapiens

<400> 7

ccgaatagga acgttgagcc gt                                                22

<210> 8
<211> 22
<212> DNA
<213> Homo Sapiens

<400> 8

gcaaatgtta tcgaggtccg gc                                                22

<210> 9
<211> 20
<212> DNA
<213> Homo Sapiens

<400> 9

acgcacaggt ggcgaatctc                                                   20

<210> 10
<211> 17
<212> DNA
<213> Homo Sapiens

<400> 10

acggtcgcga ttcggcg                                                      17

## Description

The invention relates to a method for the analysis of the methylation status of a nucleic acid according to claim 1, to an oligonucleotide according to claim 41, to a kit for the realization of these methods according to claims 42, and to the use of this method and these kits according to claims 46, 47, 48, and 49.

Throughout this application, various publications are cited. The disclosure of these publications is hereby incorporated by reference in its entirety into this application to describe more fully the state of the art to which this invention pertains.

Gene regulation has been correlated with methylation of a gene or genome. Certain cell types consistently display specific methylation patterns, and this has been shown for a number of different cell types (Adorjan et al. (2002) Tumor class prediction and discovery by micro array-based DNA methylation analysis. Nucleic Acids Res 30(5) e21).

In vertebrates, DNA is methylated nearly exclusively at cytosine bases located 5' to guanine. Such sites are being referred to as CpG dinucleotides. This modification has important regulatory effects on gene expression, especially when involving CpG rich areas, known as CpG islands, located in the promoter regions of many genes. While almost all gene-associated islands are protected from methylation on autosomal chromosomes, extensive methylation of CpG islands has been associated with transcriptional inactivation of selected imprinted genes and genes on the inactive X-chromosome of females.

Differential methylation patterns have great relevance for understanding disease and developing diagnostic applications. The identification of 5-methylcytosine within a DNA sequence is of importance in order to uncover its role in gene regulation. The position of a 5-methylcytosine cannot be identified using methods that are based on base pair behavior, since methylated cytosine behaves just as an unmethylated cytosine as per its hybridization preference.

Furthermore, in any standard amplification or sequencing reaction, this epigenetic information will be lost.

Several methods are known to solve this problem. Generally, genomic DNA is treated with a chemical or enzyme leading to a conversion of the cytosine bases, which consequently allows one to distinguish between methylated and unmethylated cytosine. The most common methods are a) the use of methylation-sensitive restriction enzymes capable of differentiating between methylated and unmethylated DNA and b) treatment with bisulfite. The use of methylation-sensitive restriction enzymes, however, is limited due to the selectivity of the restriction enzyme towards a specific recognition sequence.

In contrast, bisulfite specifically reacts with unmethylated cytosine regardless of the surrounding sequence. Upon subsequent alkaline hydrolysis, the unmethylated cytosine is converted to uracil, while 5-methylcytosine remains unmodified during this treatment (Shapiro et al. (1970) Nature 227: 1047)). Therefore, it is currently the most favored method of use for analyzing DNA for the presence of 5-methylcytosine. Uracil exhibits the same base pairing behavior as thymine; that is, it hybridizes with adenine. 5-methylcytosine does not change its chemical properties under this treatment and therefore still hybridizes with guanine. Consequently, under bisulfite treatment, the original DNA is converted in such a manner that 5-methylcytosine can now be detected as cytosine whereas those cytosines that were unmethylated in the original DNA can now be detected as uracil, showing the base pair behavior of thymine. Due to this, 5-methylcytosine, which originally could not be distinguished from cytosine by its hybridization behavior, can now be differentiated from cytosine using conventional molecular biological techniques, such as amplification and hybridization or sequencing, all of which are based on base pairing.

An overview of the further known methods of detecting 5-methylcytosine may be gathered from Fraga FM and Esteller M, Biotechniques (2002) 33(3): 632, 634, 636-649.

As the use of methylation-specific enzymes is dependent on the presence of restriction sites, most methods are based on a bisulfite treatment that is conducted before a detection or amplifying step (for review: DE 100 29 915 A1, page 2, lines 35-46 or the according translated US application 10/311,661; see also WO 2004/067545).

The term 'bisulfite treatment' is meant to comprise treatment with a bisulfite, a disulfite or a hydrogensulfite solution. As known to the expert skilled in the art and according to the invention, the term "bisulfite" is used interchangeably for "hydrogensulfite".

Several laboratory protocols are known in the art, all of which comprise of the following steps: The genomic DNA is isolated, denatured, converted several hours by a concentrated bisulfite solution, and finally desulfonated and desalted (e.g.: Frommer et al., 1992. A genomic sequencing protocol that yields a positive display of 5-methylcytosine residues in individual DNA strands. Proc Natl Acad Sci USA.; 89(5): 1827-1831).

The treatment with bisulfite (or similar chemical agents or enzymes) with the effect of altering the base pairing behavior of either methylated or unmethylated cytosine specifically, thereby introducing different hybridization properties, makes the treated DNA more applicable to the conventional methods of molecular biology, especially the polymerase-based amplification methods.

A quantification of the degree of methylation is necessary for different applications, e.g., for classifications of tumors, for prognostic information or for the prediction of drug effects. Different methods are known for the quantification of the degree of methylation, e.g. by Ms-SNuPE, by hybridizations on microarrays, by hybridization assays in solution or with by bisulfite sequencing (for review: Fraga and Estella (2002), Biotechniques 33(3): 632, 634, 636-649.). A powerful quantification tool based on real time PCR detection is the so called "QM-Assay" described in PCT/EP2005/003793.

In order to characterize the methylation patterns of different tissue types genome wide, methods are required that can detect methylation patterns by automated high throughput technologies in a reliable manner.

As briefly mentioned above, cytosine methylation plays a crucial role in several important biological processes, including embryonic development, gene regulation and disease development. The identification of methylated cytosines is therefore of major scientific interest. Of particular medical importance is the involvement of methylation in cancer development. Cancer is associated with major epigenetic changes in a variety of genes. As these changes take place at a very early stage in disease development, it is possible to use "methylation markers" (genes showing a specific methylation pattern in a particular cancer) as a diagnostic tool for early cancer detection. This approach has several crucial advantages compared to the conventional diagnostic methods, particularly with regard to a cancer detection out of bodily fluids like blood or urine: Methylation patterns of tumor-DNA released into bodily fluids can be detected early in cancer development. This is in contrast to RNA, which is chemically unstable, and to proteins, which are not amplifiable (for a review, see Laird: The power and the promise of DNA methylation markers. Nat Rev Cancer. 2003; 3(4): 253-66).

Furthermore, early detection is one of the most promising approaches to reducing the growing cancer burden. If detected early, cancer is often curable. However, the likelihood of a successful therapy of late-diagnosed patients is still poor. For example, if colorectal cancer is detected when the disease is limited to one tumor, patients are nine times as likely to survive for five years compared to those who have formed metastasis (for review, see Etzioni et al: The case for early detection. Nat Rev Cancer. 2003 Apr; 3(4):243-52).

In summary, the early detection of methylation markers in body fluids has an enormous diagnostic potential and is therefore of particular technical interest. Further important applications of methylation analysis are the molecular classification of tumours, the detection of non-cancer diseases and the prediction of a treatment response. Consequently, there is a great technical need of sensitive, specific, high-throughput and quantitative methods for the methylation analysis.

A method to detect single nucleotide polymorphisms (SNPs) has been described (Hardenbol P et al. (2003) Multiplexed genotyping with sequence-tagged molecular inversion probes. Nature Biotech 21: 6, 673-678).

The use of a method known for the detection of a single nucleotide polymorphism is, however, not easily transferable to the detection of methylation, since bisulfite treated DNA differs chemically and physically in many ways form genomic DNA, especially in length, complexity, base composition and spatial structure:
- During bisulfite treatment, the treated DNA is fragmented. The degree of the fragmentation depends on the conditions of the reaction. The longer the reaction lasts and the higher the temperature, the greater the decomposition rate of the DNA. At the same time, long reaction times are necessary for a complete transformation. Therefore, the bisulfite treated DNA is present in a complex mixture of fragments having different length and potentially incomplete transformed fragments (see: Grunau et al., 2001: Bisulfite genomic sequencing: systematic investigation of critical experimental parameters. Nucleic Acids Res. (2001); 29(13): E65-5). As the incomplete transformed DNA is a source of error for false-positive signals, it must be secured during analysis that only the transformed DNA will be detected.
- Bisulfite treated DNA contains other bases then genomic DNA, because all unmethylated cytosines are converted into uracil with bisulfite. Uracil, however, is not present in genomic DNA. Due to the high sequence repetition after bisulfite treatment, the (single-stranded) DNA potentially forms short intramolecular double-stranded segments.
- Bisulfite treated DNA contains a different base composition than genomic DNA. Genomic DNA contains four bases. In contrast, bisulfite treated DNA consists in large part only of three bases, because all non-methylated cytosines are converted to uracil. These are at least all cytosines which are located outside the sequence context CpG. As a result, bisulfite treated DNA is less complex than genomic DNA with respect to base composition, and sequence repetitions occur more often. Therefore, the design of specific primers or probes is much more difficult.
- A feature of the genomic DNA is the double helix structure. In contrast, bisulfite treated DNA is not double-stranded but single-stranded. This is due to the fact that through bisulfite treatment two non-complementary DNA strands are generated, which do not hybridize with each other. When, for example, C is transformed into U on one strand, the complementary G on the reverse strand remains unchanged. Formation of a base pair between the new generated U and the remaining G is not possible. Therefore, as a result, bisulfite treated DNA is more complex than genomic DNA with respect to its information not being present in a redundant form.

Therefore, genomic DNA and bisulfite treated DNA differ chemically and physically in various manners, rendering it impossible to use most methods used for mutation analysis also for methylation analysis.

Furthermore, the present invention relates to methods that can be used for the detection of methylated DNA in tissue sections, such as the one described by Bibikova M et al. ((2006) High-throughput DNA methylation profiling using universal bead arrays. Genome Res 16: 383-393), which allows for the determination of the methylation status of a certain CpG site using bisulfite treated DNA. This method can be used to determine the methylation status of several CpG sites simultaneously. However, four primers are needed to analyze one given CpG position. This implies that the method according to Bibikova M et al. needs to be established carefully in order to avoid any bias that might occur due to one primer pair being favored over the other in an extension and/or amplification reaction. Furthermore, the method according to Bibikova M et al. does not allow to perform a quality control for the cytosine conversion reaction using bisulfite.

Therefore, the problem underlying the present invention was to provide a method for analyzing a nucleic acid with respect to its methylation status that could be performed in a reliable and easy manner and that would also allow to perform a quality control for the conversion reaction. Furthermore, a kit was to be supplied with which the method according to the present invention could be realized.

Surprisingly, the inventors were able to solve this problem by inventing the present method. The central idea of the invention is to provide a molecule that undergoes a structural change if a certain methylation status is present in the nucleic acid to be analyzed. This structural change can then be detected.

This newly developed method allows for the analysis of a single methylation site or of a multitude of methylation sites simultaneously.

### Description of the invention

Disclosed is a method for the analysis of the methylation status of a nucleic acid.

According to the method of the present invention, at least the following four steps will be performed, preferably in the given order.
First, a template DNA that is to be analyzed with respect to its methylation status is treated with at least one chemical reagent or with at least one solution containing at least one enzyme, whereby the base pairing behavior of methylated cytosine bases and/or unmethylated cytosine bases of the nucleic acid are altered such that methylated cytosine bases become distinguishable from unmethylated cytosine bases in terms of their hybridization properties, i.e. base pairing properties.
Secondly, an at least one linear oligonucleotide that is ligatable to itself if a particular methylation status is present (or was present before treatment) at a particular site in the nucleic acid to be analyzed, is hybridized to the treated nucleic acid. In order to be ligatable, the at least one linear oligonucleotide contains a phosphate group at its 5' end.
Thirdly, the at least one oligonucleotide is ligated to itself (i.e. the 5' end of the at least one oligonucleotide is ligated to the 3' end at least one oligonucleotide) to form a circular DNA molecule. The at least one oligonucleotide is designed such that this ligation occurs only if a particular methylation status is present (or was present before treatment) in the nucleic acid to be analyzed. Therefore, the formation of a circular DNA molecule is indicative of a certain methylation status of the nucleic acid to be analyzed.
Fourth, the DNA molecule formed is amplified to detect the methylation status of the nucleic acid, i.e. whether the nucleic acid was methylated and/or unmethylated. As will be explained below in greater detail, the DNA molecule can still be circular when the amplification step is performed, or can be performed on a linear molecule. In the latter case, a cleavage reaction is performed after the ligation but prior to the amplification reaction.

The method according to the present invention is useful for analyzing the methylation status of nucleic acid samples.

The method is further advantageous, because it can be successfully used on short nucleic acid molecules, particularly those nucleic acid molecules that have been partially degraded. Such degraded nucleic acids can be found in body fluid or paraffin tissue samples.

The method according to the present invention can be used to analyze a nucleic acid that contains a first sequence portion and a second sequence portion which are located adjacent to each other. The term "adjacent" is meant such that the first sequence portion and the second sequence portion are not more than ten nucleotides apart from each other. Preferably, only one CpG site is located between the first sequence portion and the second sequence portion of the nucleic acid to be analyzed. Most preferably, the first and the second sequence portion of the nucleic acid are only one nucleotide apart.

The at least one oligonucleotide that hybridizes to such a treated nucleic acid comprises a first sequence portion for hybridizing to the first sequence portion of the treated nucleic acid, a second sequence portion for hybridizing to the second sequence portion of the treated nucleic acid, and a third sequence portion which is located between the first and the second sequence portion of the at least one oligonucleotide and which allows for the at least one oligonucleotide to form a circle-like structure when the first and the second sequence portion of the at least one oligonucleotide are hybridized to the first and the second sequence portion of the treated nucleic acid.

The first sequence portion and/or the second sequence portion are each between 5 nucleotides (nt) and 50 nt in length, preferably between 10 nucleotides (nt) and 30 nt in length, and most preferably between 15 nt and 18 nt in length. The third sequence portion is between 15 nt and 50 nt in length, preferably between 25 nt and 40 nt in length, and most preferably between 30 nt and 36 nt in length.

After the treatment step of the present invention has been performed, the present method can proceed in two general embodiments with regard to hybridizing an at least one linear oligonucleotide that is ligatable to itself to the treated nucleic acid:
1. A first embodiment the present method can be used to analyze a nucleic acid in which the first and the second sequence portion are directly beside each other, without a gap between them.
2. In a second embodiment of the present method, the method can be used to analyze a nucleic acid in which the first and the second sequence portion are separated by a gap of at least one oligonucleotide. According to this embodiment of the present invention, it is a nucleotide within this gap that can be analyzed with regard to its methylation status (or with regard to the methylation status that was present before the treatment that altered the base pairing behavior).

Following the hybridization step, a gap that might be present between the ends of the at least one oligonucleotide will be extended dependent on the methylation status of the nucleic acid to be analyzed.

For both the first and the second embodiment of the present invention, i.e. independent from the presence of a gap between the first and the second sequence portion of the nucleic acid to be analyzed, a ligation reaction is performed, possibly resulting in a circular DNA molecule.

This circular DNA molecule can then either be directly amplified or cleaved prior to amplification. Different amplification methods can be used, such as rolling circle amplification (RCA) or polymerase chain reaction (PCR).

Each of these methods for amplification can than be combined with any suited detection method, such as detection by microarray or length polymorphism.

Now different embodiments with regard to the hybridization step of the at least one oligonucleotide to the nucleic acid to be analyzed will be described.

### Hybridization without the formation of a gap

When the first sequence portion and the second sequence portion of the treated nucleic acid to be analyzed do not have a gap between them, the first and/or the second sequence portion of the at least one oligonucleotide preferably comprises at least one first base for the analysis of a methylated cytosine base which hybridizes to a treated methylated cytosine base and/or at least one second base for the analysis of an unmethylated cytosine base which hybridizes to a treated unmethylated cytosine base. Preferably, this at least one first base is located near or at the end of the linear at least one oligonucleotide, because this allows easy differentiation between a perfectly hybridized oligonucleotide and an oligonucleotide with a mismatch.

Another advantage of the method according to this embodiment of the present invention is that it can also be used to verify that the treatment reaction of the nucleic acid occurred to completion. A complete conversion of all the methylated and/or unmethylated cytosine bases is a prerequisite for the correct analysis of the methylation status of the nucleic acid and therefore needs to be monitored.

When the first and the second sequence portion of the at least one oligonucleotide are both reverse complementary to the first and to the second sequence portion of the treated nucleic acid so that there is no mismatch between the first sequence portion of the at least one nucleotide and the first sequence portion of the treated nucleic acid on the one hand and the second sequence portion of the at least one nucleotide and the second sequence portion of the treated nucleic acid on the other hand, the at least one oligonucleotide forms a circle-like structure, whereby the 5' end of the at least one oligonucleotide is positioned directly at the 3' end of the at least one oligonucleotide, rendering the at least one oligonucleotide ligatable. The first sequence portion of the hybridized at least one oligonucleotide is then ligated to the second sequence portion of the hybridized at least one oligonucleotide to form a circular DNA molecule.

The nucleic acid to be analyzed is usually a genomic nucleic acid, since the epigenetic information of methylation is only found in this type of a nucleic acid. The genomic DNA may be isolated and/or denatured and therefore present as single strands. It is, however, also possible to use a nucleic acid from other sources such as synthesized DNA that does not stem from a natural source and which might have been methylated in vitro or synthesized using methylated cytosine bases.

### Hybridization with the formation of a gap

In another embodiment, the method according to the present invention can be used on a nucleic acid, wherein the first and the second sequence portion of the treated nucleic acid are located adjacent to each other, with a gap of up to ten nucleotides, preferably of one nucleotide between them. This gap comprises or preferably is formed by a nucleotide which is (or was before treatment) a methylated or unmethylated cytosine, the methylation status of which is to be analyzed. Additionally, the first sequence portion of the at least one oligonucleotide is reverse complementary to the first sequence portion of the treated nucleic acid, and the second sequence portion of the at least one oligonucleotide is reverse complementary to the second sequence portion of the treated nucleic acid. Therefore, in contrast to the embodiment without a gap as described above, here the first and second portion of the at least one oligonucleotide are not used to analyze the nucleic acid. Instead, the analysis is performed base on the sequence gap.

### Extension reaction

When a gap is present between the first and the second sequence portion of the nucleic acid, an extension reaction is performed prior to ligating the first sequence portion of the hybridized at least one oligonucleotide to the second sequence portion of the hybridized at least one oligonucleotide. In this extension reaction the nucleotide gap between the first and the second sequence portion of the at least one oligonucleotide is filled so that the ends of the at least one oligonucleotide can be ligated to each other. The extension reaction is performed with either a first deoxyribonucleotide (dNTP), which forms a base pair with a treated methylated cytosine base to form a circular DNA molecule if the nucleic acid to be analyzed was methylated at the position of the gap, or with a second deoxyribonucleotide (dNTP), which forms a base pair with a treated unmethylated cytosine base to form a circular DNA molecule if the nucleic acid to be analyzed was unmethylated at the position of the gap. In order to be able to differentiate whether the nucleic acid was methylated or unmethylated at the position of the gap, the first and the second deoxyribonucleotide are different from each other.

The extension reaction is performed using a polymerase, preferably a thermostable polymerase. The ligation reaction is preferably performed using a ligase, preferably a thermostable ligase.

As a control, an additional extension reaction can be performed with a third deoxyribonucleotide which does not form a base pair with neither a treated methylated cytosine base nor with a treated unmethylated cytosine base. The signal obtained from this third deoxyribonucleotide, which should not be introduced into the at least one oligonucleotide, yields a background signal. The data obtained for the methylated and/or unmethylated position of the nucleic acid can be corrected using this control signal.

### Bisulfite conversion reaction

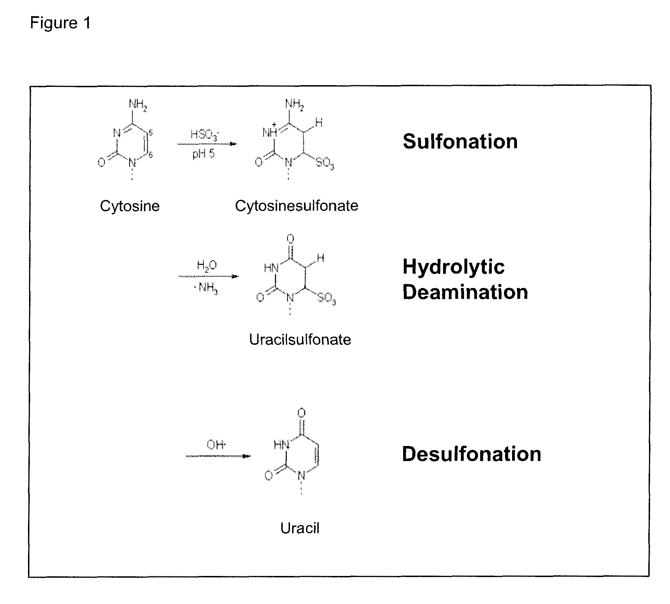

In a preferred embodiment of the present invention, the nucleic acid to be analyzed is treated with a bisulfite solution containing a bisulfite reagent, whereby unmethylated cytosine bases of the nucleic acid are converted into uracil bases while 5-methylcytosine bases remain unchanged. The chemical reaction occurring will later be described with reference to figure 1. The term "bisulfite reagent" refers to a reagent comprising bisulfite, disulfite, hydrogen sulfite or combinations thereof, useful to distinguish between methylated and unmethylated CpG dinucleotide sequences. Methods to perform this treatment are known in the art (see e.g. PCT/EP 2004/011715).

It is preferred that the bisulfite treatment is conducted in the presence of denaturing solvents, such as, but not limited to, n-alkylenglycol, particularly diethylene glycol dimethyl ether (DME), or in the presence of dioxane or dioxane derivatives. In a preferred embodiment, the denaturing solvents are used in concentrations between 1 % and 35 % (v/v). It is also preferred that the bisulfite reaction is carried out in the presence of scavengers such as, but not limited to, chromane derivatives, e.g. 6-hydroxy-2,5,7,8-tetramethylchromane 2-carboxylic acid or trihydroxybenzoe acid and derivates thereof, e.g. Gallic acid (see: PCT/EP2004/011715). The bisulfite conversion is preferably carried out at a reaction temperature between 30 °C and 70 °C, whereby the temperature is increased to over 85 °C for short periods of times during the reaction (see: PCT/EP2004/011715). The bisulfite-treated DNA is preferably purified prior to further treatment. This may be conducted by any means known in the art, such as, but not limited to, ultrafiltration, preferably carried out by means of Microcon™ columns (manufactured by Millipore™). The purification is carried out according to a modified manufacturer's protocol (see: PCT/EP2004/011715).

It is also preferred that the bisulfite reaction is carried out in the presence of scavengers such as but not limited to chromane derivatives, e.g. 6-hydroxy-2,5,7,8-tetramethylchromane 2-carboxylic acid or trihydroxybenzoe acid and derivates thereof, e.g. Gallic acid (see: PCT/EP2004/011715 which is incorporated by reference in its entirety). The bisulfite conversion is preferably carried out at a reaction temperature between 30 °C and 70 °C, whereby the temperature is increased to over 85 °C for short periods of times during the reaction (see: PCT/EP2004/011715).

Instead of a chemical conversion, it is furthermore possible to conduct the conversion enzymatically, e.g. by use of methylation-specific cytidine deaminases (see DE 103 31 107 B; PCT/EP2004/007052).

When the nucleic acid is treated with bisulfite and no gap is present between the first and the second sequence of the nucleic acid, the first and/or the second sequence portion of the at least one oligonucleotide comprise(s) in a preferred embodiment at least one guanine base as an at least one first base for the analysis of a methylated cytosine base which forms a base pair with (i.e. hybridizes to) a treated methylated cytosine base and/or at least one thymine base as an at least one second base for the analysis of an unmethylated cytosine base which forms a base pair with (i.e. hybridizes to) a treated unmethylated cytosine base.

### Extension reaction with bisulfite treated nucleic acid

When performing an extension reaction to fill a gap between the 5' and the 3' end of the at least on oligonucleotide with a bisulfite treated nucleic acid as a template, the first deoxyribonucleotide is preferably dGTP, and the second deoxyribonucleotide is preferably dATP. dGTP is only added to the 3' end of at least one oligonucleotide when the nucleotide of the treated nucleic acid between the first and the second sequence portion is a methylated cytidine and therefore was chemically changed during the bisulfite treatment. dATP is only added to the 3' end of at least one oligonucleotide by a polymerase when the nucleotide of the treated nucleic acid between the first and the second sequence portion was an unmethylated cytidine which was converted to uridine during the bisulfite treatment, because uridine forms a base pair with adenosine.

The third deoxyribonucleotide which is to be used in the control extension reaction is dCTP or dTTP, both of which do not form base pairs with either a cytidine or uridine nucleoside.

### Exonuclease reaction

After the ligation reaction has been completed, at least one exonuclease is preferably added to the reaction mixture to digest all the at least one oligonucleotides that were not ligated. Since the at least one oligonucleotide is usually added in access to the number of given targets in a sample, this step reduces the amount of unspecific amplification products and therefore reduces the danger of false results.

### Amplification reaction

For convenient amplification of the DNA molecule that is formed in the ligation step of the at least one oligonucleotide to itself, the third sequence portion of the at least one oligonucleotide comprises a first primer sequence to which a first primer can bind. This first primer can for example be used in a rolling circle amplification, for which one primer is sufficient. The advantage of a rolling circle amplification is that the resulting signal is linear and not exponential in nature, which can make interpretation and quantitation of the data obtained more easy.

If, however, an exponential amplification of the at least one oligonucleotide is preferred, the third sequence portion of the at least one oligonucleotide further comprises a second primer sequence to which a second primer can bind. This second primer can then together with the first primer be used in an exponential amplification, such as the polymerase chain reaction (PCR). Preferably, the polymerase chain reaction is a real-time polymerase chain reaction.

As an amplification reaction, a polymerase-based amplification reaction is preferred, in particular a polymerase chain reaction, which is preferably performed using a heat stable polymerase. However, it is also possible to apply other enzymatic amplification reactions known to the person skilled in the art.

Preferably, the first and the second primer sequences are oriented such that the DNA molecule formed can be amplified only if the first sequence portion of the at least one oligonucleotide was ligated to the second sequence portion of the at least one oligonucleotide.

### Components of the at least one oligonucleotide

In a preferred embodiment of the method according to the invention, the third sequence portion of the at least one oligonucleotide comprises a first cleavage site for cleaving the at least one oligonucleotide for releasing the at least one oligonucleotide from the treated nucleic acid. Such a cleavage site can be formed by at least one uracil base within the third sequence portion of the at least one oligonucleotide, which is removable by uracil-N-glycosylase (UNG). Preferably, three to four uracil base are present within the third sequence portion of the at least one oligonucleotide. Consecutively, the at least one oligonucleotide can be treated with uracil-N-glycosylase to remove the at least one uracil base and to create at least one abasic site. Upon heating, the at least one oligonucleotide will be cleaved at the abasic site, which causes the release of the at least one oligonucleotide from the treated nucleic acid, because the number of hybridizing base pairs has been reduced. It is, however, also possible to use a restriction site for a restriction enzyme as a first cleavage site.

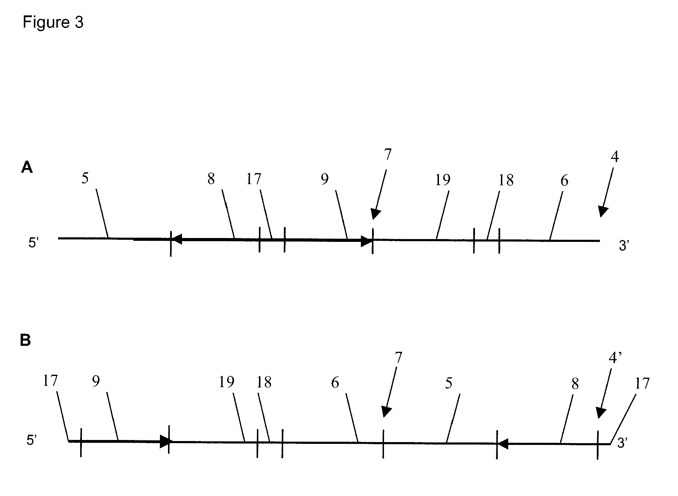

In addition to the first cleavage site, the third sequence portion of the at least one oligonucleotide preferably further comprises a second cleavage site for cleaving the at least one oligonucleotide for making the tag sequence accessible, so that the cleaved at least one oligonucleotide can hybridize via the tag to an oligonucleotide that is immobilized on a substrate. In this cleavage reaction, the first and the second sequence portions of the at least one oligonucleotide are preferably removed. Therefore, the second cleavage site should be located close to one end of the tag sequence, preferably between the tag sequence on the one side and the first and second sequence portions of the at least one oligonucleotide on the other side (see also the description with reference to figure 3). The second cleavage site is preferably a restriction site, which is recognized and cleaved by a suitable restriction enzyme.

### Detection of the methylation status

When the detection of the methylation status of the nucleic acid is detected by different means than a polymerase based amplification, a chip-based detection can be performed. In this case, the third sequence portion of the at least one oligonucleotide comprises a tag sequence for hybridizing to an oligonucleotide that is immobilized on a solid substrate, preferably on a biochip. Such biochips are known to a person skilled in the art.

The tag sequence is between 5 nucleotides (nt) to 50 nt long, preferably between 10 nt to 40 nt long, most preferably between 16 nt to 19 nt long or most preferably between 20 nt to 25 nt long.

If the amplification products are to be detected using a chip, then the amplified DNA molecules formed are labeled, namely either with a first dye, if the extension reaction was performed with the first deoxyribonucleotide, or a second dye, if the extension reaction was performed with the second deoxyribonucleotide. Both the first and the second dye can each generate a detectable signal, and the signal from the first dye is distinguishable from the signal of the second dye. Thereby, conclusions about the methylation status of the nucleic acid can be drawn.

A chip is defined here as an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescence labeled probes are often used for the scanning of immobilized DNA arrays. The simple attachment of Cy3 and Cy5 dyes to the 5'-OH of the specific probe is particularly suitable for fluorescence labels. The detection of the fluorescence of the hybridized probes may be carried out, for example, via a confocal microscope. Cy3 and Cy5 dyes, besides many others, are commercially available.

Furthermore, if the extension reaction was performed with the third deoxyribonucleotide used as a control, the amplified DNA molecule formed is furthermore labeled with a third dye, to detect a background signal that can be subtracted from the signals stemming from the first and/or second dye, which are separately detected. Therefore, the sample that is labeled with the third dye can be referred to as in (internal) standard for background correction.

The labeled amplified DNA molecule formed are then hybridized to the molecules immobilized on a substrate, e.g. a microarray chip. Preferably, the chip has oligonucleotides spotted on it that are able to interact with the tag sequence of the amplificates of the at least one oligonucleotide.

### Multiplexing

If the method according to the present invention can also be used to analyze the nucleic acid at different sequence sites simultaneously. For this purpose, a multitude of the at least one oligonucleotide, which differ in their first and second sequence portions from each other, is used. The multitude of the at least one oligonucleotide differs in unique first and unique second sequence portions to detect different possible methylation sites. In addition, the multitude of the at least one oligonucleotide also has a unique tag sequence for each target methylation site. Universal to all of the at least one oligonucleotides of the multitude is the first and second primer sequences and the first and second cleavage sites.

The multitude of the at least one oligonucleotide comprises 2 to 2,000 different oligonucleotides, preferably 5 to 500, most preferably 50 to 200 different oligonucleotides.

The multitude of tag sequences needs to fulfill the following criteria to achieve high hybridization specificity and to be best suited for a multiplex usage: The tag sequence should be designed such that the range of melting temperatures is narrow. Also, sequences forming secondary structures should be avoided. If possible, the tag sequences chosen should not be similar to sequences of the organism from which the nucleic acid to be analyzed stems from. This way, unspecific hybridization is limited.

Before the formed amplified DNA molecules are hybridized to the microarray, the labeled DNA molecules are combined. Relative intensities of the first and second dye, preferably corrected by subtracting the background signal stemming from the third dye, indicate the methylation status of a particular target methylation site of the analyzed nucleic acid.

If a chip is used for quantitative signal detection, the labeled amplified DNA molecule formed is hybridized with nucleic acids (oligonucleotides, cDNA, etc.) immobilized on a microarray (chip, oligonucleotides on support).

Binding of the amplificates to the molecules immobilized on a microarray is done using a tag sequence for hybridizing to an oligonucleotide that is immobilized on a substrate. This tag sequence is a sequence unique for each CpG site analyzed, if several CpG sites are analyzed simultaneously. In such a case, the molecules immobilized on a chip comprise a sequence that is reverse complementary to the tag sequence which allows them to bind to an oligonucleotide comprising a tag sequence. Since the position of the immobilized molecules on the chip in correlated to their sequence, a signal coming from a particular position on the chip can be allotted to a certain sequence.

Based on the signals that were detected, a ratio can be calculated based the signals stemming from a methylated and an unmethylated site.

The detection of the signal can be performed using a chip, in particular a microarray, as described above, or using another suited method, such as methylation specific PCR (MSP) (Herman JG, Graff JR, Myohanen S, Nelkin BD and Baylin SB. (1996), Methylation-specific PCR: a novel PCR assay for methylation status of CpG islands. Proc Natl Acad Sci USA 93: 9821-9826), MethyLight® (US 6,331,393 B1), or heavy methyl (HM) (WO 02/072880; Cottrell SE et al. Nucleic Acids Res 2004; 32(1): e10).

The problem underlying the present invention is also solved by an oligonucleotide for analyzing the methylation status of a nucleic acid with a first and second sequence portion which are located adjacent to each other. Such an oligonucleotide comprises a first sequence portion for hybridizing to the first sequence portion of the treated nucleic acid, a second sequence portion for hybridizing to the second sequence portion of the treated nucleic acid, and a third sequence portion which is located between the first and the second sequence portion of the at least one oligonucleotide and which allows for the at least one oligonucleotide to form a circle-like structure when the first and the second sequence portion of the at least one oligonucleotide are hybridized to the first and the second sequence portion of the treated nucleic acid.

According to the invention, such an oligonucleotide contains only adenine, cytosine, and thymine, but no guanine base in its first and the second sequence portion that hybridize to the first and second sequence portion of the treated nucleic acid, if the first and the second sequence portion of the nucleic acid to be analyzed were completely unmethylated.

Such an oligonucleotide can be used if the first and the second sequence portion of the nucleic acid are separated by a nucleotide gap of at least one nucleotide, preferably of one nucleotide.

Preferred embodiments of such an oligonucleotide are described above with reference to the method according to the present invention.

The problem underlying the present invention is also solved by a kit suitable for performing the method according to the present invention as described above. Such a kit comprises a) a chemical reagent or an enzyme for treating a nucleic acid, which alters the base pairing behavior of methylated cytosine bases and/or unmethylated cytosine bases of the nucleic acid such that methylated cytosine bases become distinguishable from unmethylated cytosine bases, preferably bisulfite, b) at least one oligonucleotide that is ligatable to itself, and c) an enzymatic activity for ligating the at least one oligonucleotide and/or an enzymatic activity for amplifying a nucleic acid.

Furthermore, it is preferred that the at least one oligonucleotide in the kit comprises
- a first sequence portion for hybridizing to a first sequence portion of a treated nucleic acid,
- a second sequence portion for hybridizing to a second sequence portion of the treated nucleic acid, and
- a third sequence portion which is located between the first and the second sequence portion and which allows for the at least one oligonucleotide to form a circle-like structure when hybridized to the treated nucleic acid.

Preferred is a kit in which the at least one oligonucleotide is the oligonucleotide described above.

Further characteristics of the at least one oligonucleotide which is ligatable to itself as part of the kit were described above with reference to the method of the present invention.

The at least one oligonucleotide of the test kit enables the correct and simple determination of the methylation status by introducing an oligonucleotide that interacts with a treated nucleic acid in which methylated cytosines are distinguishable from unmethylated cytosines to undergo a structural change through ligation if the treated nucleic acid is present in a certain methylation status. The properties of the at least one oligonucleotide are the same as described above with reference to the method according to the present invention.

The described test kit may further comprise at least one of the additional components:
- a denaturing reagent and/or solution, for example: dioxane or diethylene glycol dimethylether (DME) or any substance, which is suitable as described in WO 05/038051,
- a scavenger, for example 6-hydroxy-2,5,7,8-tetramethylchromane 2-carboxylic acid or other scavengers as described in WO 01/98528 or WO 05/038051,
- at least one additional primer, which is suitable for the amplification of one or more DNA amplificates,
- a reaction buffer, which is suitable for a bisulfite treatment and/or a PCR reaction,
- nucleotides, which can be dATP, dCTP, dTTG, dUTP and dGTP or any derivative of these nucleotides, including bases with altered pairing properties ("wobbles"),
- MgCl₂ as a substance or in solution and/or any other magnesium salt, which can be used to carry out a DNA polymerase replication,
- a DNA polymerase, for example Taq polymerase or any other polymerase with or without proof-reading activity,
- any reagent, solution, device and/or instruction which is useful for realization of a method according to the invention.

The problem underlying the present invention is also solved by the use of the method as described above or of an oligonucleotide as described above or of a kit as described above for diagnosis and/or prognosis of adverse events for patients or individuals, whereby diagnosis means diagnose of an adverse event, a predisposition for an adverse event and/or a progression of an adverse event.

These adverse events belong to at least one of the following categories: undesired drug interactions; cancer diseases; CNS malfunctions; damage or disease; symptoms of aggression or behavioral disturbances; clinical; psychological and social consequences of brain damages; psychotic disturbances and personality disorders; dementia and/or associated syndromes; cardiovascular disease, malfunction or damage; malfunction, damage or disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and metabolic malfunction, damage or disease; headaches or sexual malfunction.

The problem underlying the present invention is furthermore solved by the use of the method as described above or of an oligonucleotide as described above or of a kit as described above for distinguishing cell types and/or tissues and/or for investigating cell differentiation.

The problem underlying the present invention is furthermore solved by the use of the method as described above or of an oligonucleotide as described above or of a kit as described above for identifying an indication-specific target in a nucleic acid, which is defined by a difference in the methylation of a nucleic acid derived from a diseased tissue in comparison to the methylation of a nucleic acid derived from a healthy tissue.

The method and test kits also serve for distinguishing cell types or tissues or for investigating cell differentiation. They also serve for analyzing the response of a patient to a drug treatment.

The method and test kit of the invention can also be used to determine the DNA methylation status in that positions are methylated or non-methylated compared to normal conditions if a single defined disease exists. In a particular preferred manner they can serve for identifying an indication-specific target, wherein a template nucleic acid is treated according to the method of the present invention, and wherein an indication-specific target is defined as differences in the DNA methylation status of a DNA derived from a diseased tissue in comparison to a DNA derived from a healthy tissue. The tissue samples can originate from a patient with the single defined disease and from a healthy individual. They can also originate from only one patient suffering from the single defined disease, in which case DNA from the pathological tissue will be compared to DNA from healthy tissue that was obtained from adjacent to the sick tissue of the patient (so-called adjacent analogous normal tissue).

In other words, DNA stemming from a healthy individual and an individual with a single defined disease will be analyzed with respect to its methylation status at particular CpG sites. The individual results are compared to each other with the goal of identifying CpG positions in genomic DNA that allow for the diagnosis of the single defined disease in a patient and/or that allow for the prediction of likelihood of an individual becoming ill with the single defined disease and/or that allow for the prediction of likelihood of an individual surviving the single defined disease.

In a particular preferred manner, the method and test kit of the invention can serve for identifying an indication-specific target, wherein a template nucleic acid is treated according to the method of the present invention, and wherein an indication-specific target is defined as differences in the DNA of a DNA derived from a diseased tissue in comparison to a DNA derived from a healthy tissue. These tissue samples can originate from diseased or healthy patients or from diseased or healthy adjacent tissue of the same patient.

The sample nucleic acid can be obtained from serum or other body fluids of an individual. They can, in particular, be obtained from cell lines, tissue embedded in paraffin, such as tissue from eyes, intestine, kidneys, brain, heart, prostate, lungs, breast or liver, histological slides, body fluids and all possible combinations thereof.

The term body fluids is meant to comprise fluids such as whole blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile, stool and cerebrospinal fluid. It is especially preferred that said body fluids are whole blood, blood plasma, blood serum, urine, stool, ejaculate, bronchial lavage, vaginal fluid and nipple aspirate fluid.

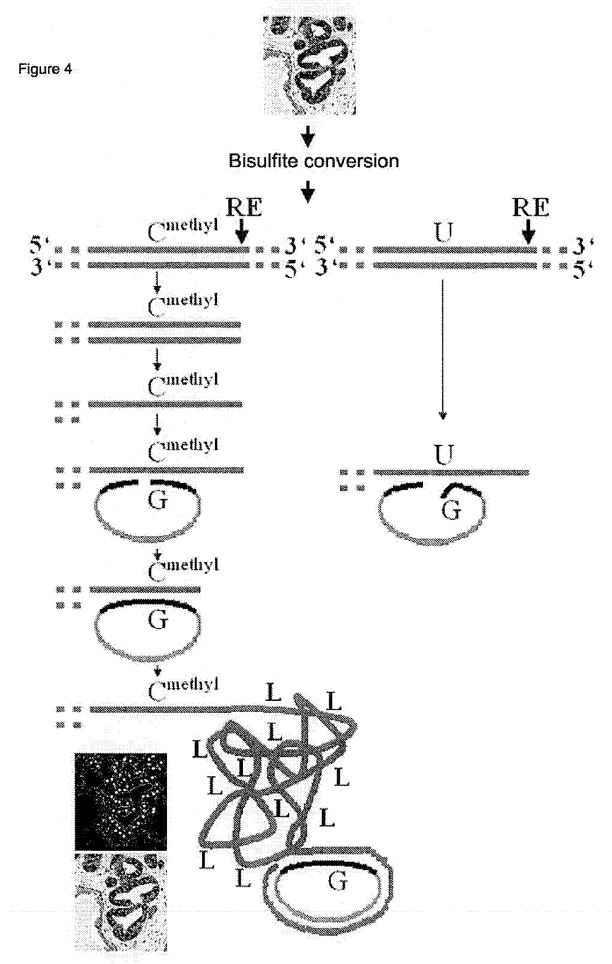

The problem underlying the present invention is furthermore solved by the use of the method as described above or of an oligonucleotide as described above or of a kit as described above for in situ diagnostics performed on a histological section, as described with reference to figure 4.

The histological section can be both fresh material as well as a paraffin-embedded, formalin-fixed section. The section can contain one or more cells, including tissue.

The generated signal is detected by microscopy, in particular by confocal microscopy. Appropriate signals can be obtained using methods of histochemistry, immunohistochemistry, pathology, histology, cell culture staining, cell biology analysis, flow through cytometry (FACS), optical imaging, and ELISA, all of which are know to a person skilled in the art.

Ways of performing in situ diagnostics are known to a person skilled in the art (see e.g., Nuovo GJ (2004) Methylation-specific PCR in situ hybridization. Methods Mol Biol 287: 261-272).

The present invention can furthermore be used to determine methylation patterns of cells and tissues, both healthy and sick, reflecting populations of (genomic) nucleic acids at various methylation sites. Put differently, it can be used to determine to what percentage a certain CpG position of a nucleic acid population has been methylated.

### List of Reference Signs

- 1: a (treated) nucleic acid to be analyzed
- 2: a first sequence portion of the (treated) nucleic acid to be analyzed
- 3: a second sequence portion of the (treated) nucleic acid to be analyzed
- 4: an at least one oligonucleotide
- 4': at least one rearranged oligonucleotide
- 5: a first sequence portion of the at least one oligonucleotide
- 6: a second sequence portion of the at least one oligonucleotide
- 7: a third sequence portion of the at least one oligonucleotide
- 8: a first primer sequence
- 9: a second primer sequence
- 10: a first primer
- 11: a second primer
- 12: a zymogen sequence portion
- 13: circular DNA molecule formed
- 14: an amplification product
- 15: a catalytically active sequence portion
- 16: a substrate
- 17: a first cleavage site of the at least one oligonucleotide
- 18: a second cleavage site of the at least one oligonucleotide
- 19: a tag sequence for binding to an immobilized nucleic acid

### Description of the drawings

### Figure 1:

Figure 1 describes the complete conversion of unmethylated cytosine to uracil, also referred to as bisulfite conversion, which is known in the art. In the first step of this reaction, unmethylated cytosine bases are sulfonated at position C6 of the ring at a pH around 5 through reaction with hydrogensulfite.

The second step of the conversion is the deamination that takes place rather spontaneously in an aqueous solution. Thereby, cytosine sulfonate reacts to uracil sulfonate. The third step of the conversion is the desulfonation step, which takes place in alkaline conditions, resulting in uracil.

### Figure 2:

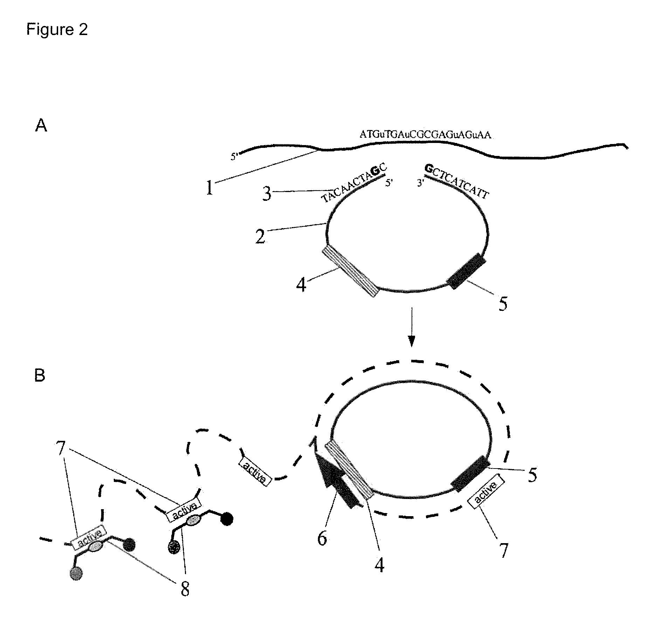

Figure 2 shows an embodiment of the method according to the present invention, in which a first and a second sequence portion 5, 6 of the at least one oligonucleotide 4 are located directly next to each other (are not separated by a gap) when hybridized to the nucleic acid 1 to be analyzed.

Part A of figure 2 depicts a single stranded nucleic acid 1 in the form of a DNA molecule 1, that was treated with bisulfite. The treated nucleic acid 1 comprises a first sequence portion 2 and a second sequence portion 3. The first and the second sequence portion 2, 3 of the treated nucleic acid 1 contain the potential methylation sites that are to be analyzed.

This DNA molecule 1 is to be analyzed with respect to its methylation status. Due to the treatment with bisulfite, the DNA molecule 1 is single stranded. Furthermore due to bisulfite treatment, all unmethylated cytosine bases of the DNA molecule 1 have been converted into uracil bases and are represented by a "u" in the nucleic acid sequence (ATGuTGAuCGCGAGuAGUAA, SEQ ID NO. 1) shown.

The at least one oligonucleotide 4, which is ligatable to itself, comprises a first sequence portion 5 for hybridizing to the first sequence portion 2 of the treated nucleic acid 1, a second sequence portion 6 for hybridizing to the second sequence portion 3 of the treated nucleic acid 1, and a third sequence portion 7 which is located between the first and the second sequence portion 5, 6 of the at least one oligonucleotide 4 and which allows for the at least one oligonucleotide 4 to form a circle-like structure when the first and the second sequence portion 5, 6 of the at least one oligonucleotide 4 are hybridized to the first and the second sequence portion 2, 3 of the treated nucleic acid 1, respectively.

In addition, the at least one oligonucleotide 4 comprises a first primer sequence 8 for binding of a first primer 10, which is used for initiating a rolling circle amplification, and a zymogen sequence portion 12, which encodes for a catalytically active nucleic acid, such as a 10-23 DNAzyme.

In the example shown, the first and the second sequence portion 5, 6 of the at least one oligonucleotide 4 each contain one guanine base, each of which is located at a position that corresponds to the CpG position to be analyzed of the treated nucleic acid 1. Specifically, the first sequence portion 5 of the at least one oligonucleotide 4 contains the sequence CGATCAACAT (SEQ ID NO. 2), and the second sequence portion 6 of the at least one oligonucleotide 4 contains the sequence TTACTACTCG (SEQ ID NO. 3).

A complete hybridization between the first sequence portion 5 of the at least one oligonucleotide 4 with the first sequence portion 2 of the treated nucleic acid 1 and between the second sequence portion 6 of the at least one oligonucleotide 4 with the second sequence portion 3 of the treated nucleic acid 1 is only possible if these positions were previously methylated in the nucleic acid 1, and therefore were not chemically modified during the treatment with bisulfite. In other words, ligation of the ends of the at least one oligonucleotide 4 will only occur if all the cytosine bases of the DNA molecule 1 were all converted into uracil during bisulfite treatment (because they were unmethylated before bisulfite treatment), except for the two centrally located cytosine bases that are each part of a CpG site, which were methylated before bisulfite treatment. If perfect hybridization occurs, the ends can be ligated to form a circular DNA molecule in a reaction that is catalyzed by a (preferably thermostable) ligase.

It is also possible to use an at least one oligonucleotide that contains an adenosine instead of a guanosine at these positions, in which case a circular DNA molecule could only be formed through ligation if the corresponding positions were unmethylated cytosines in the nucleic acid prior to bisulfite treatment, and were converted into uracil during bisulfite treatment, which forms a base pair with adenine.

In part B of figure 2, the amplification reaction of the circular DNA molecule formed 13 is shown. Here, a first primer 10 is used to initiate a rolling circle amplification (RCA) by binding to a first primer sequence portion 8. The amplification product 14 is a concatamer of the circular DNA template molecule 13, which comprises a multitude of catalytically active sequence portions 15 in the form of DNAzymes. The catalytically active sequence portion 15 has the reverse complimentary sequence of the zymogen sequence portion 12.

A substrate 16 can bind to the catalytically active sequence portion 15 through hybridization. The catalytically active sequence portion 15 can then chemically modify the substrate 8 so that it becomes detectible. The substrate 16 is preferably an oligonucleotide, in particular a tagged nucleic acid, whereby the tag is preferably a fluorescence dye. Advantageously, the tagged nucleic acid 16 is a DNA-RNA-chimera, which is tagged at one end with a fluorescence dye and at the other end with a quencher.

The substrate 16 shown comprises at one end a fluorescence dye and at the other end a quencher. Following the catalytic cleavage of the substrate 16 which is catalyzed by the catalytically active sequence portion 15, fluorescence dye and quencher are being separated from each other. Therefore, the fluorescence of the fluorescence dye becomes detectable.

The amplification product 14 comprises with increasing reaction time an increasing number of catalytically active sequence portions 15. Therefore, the overall activity of the catalytically active sequence portions 15 is also increasing with time. When performing the amplification reaction using only a first primer 10, the number of the catalytically active sequence portions 15 is increasing in a linear fashion with time.

When performing the amplification reaction using both a first primer 10 and a second primer (not shown in this figure), the number of the catalytically active sequence portions 15 is increasing exponentially with time. Accordingly, when performing the amplification reaction using only a first primer 10, the activity of the catalytically active sequence portions 15 is increasing in a linear fashion with time. When performing the amplification reaction using both a first primer 10 and a (not shown) second primer, the activity of the catalytically active sequence portions 15 is increasing exponentially with time. For both amplification modes, the detection of the signal allows to draw conclusions about the methylation of the nucleic acid 1 to be analyzed.

The amplification of the at least one oligonucleotide 4 using two primers after a cleavage reaction of the circular oligonucleotide 4 formed will later be described with reference to figure 3.

It is noted that it is also possible to use at least two oligonucleotides that differ in their number of nucleotides to analyze the methylation status. The detection of the methylation status can be performed accordingly making use of a length polymorphism, whereby the amplification products are separated according to their lengths. The abundance of an amplification product of a certain length allows to draw conclusions about the methylation status.

### Figure 3:

Figure 3 shows a schematic representation of an at least one oligonucleotide 4 before (panel A) and after (panel B) a structural change that occurs according to the method of the present invention if a certain methylation status is present in a (not shown) nucleic acid which is to be analyzed. This structural change can then be detected through or after amplification of the structurally changed at least one oligonucleotide 4.

With reference to panel A of figure 3, the at least one oligonucleotide 4 comprises a first sequence portion 5 and a second sequence portion 6, both located at the ends of the at least one oligonucleotide 4. The first sequence portion 5 and the second sequence portion 6 are chosen such that they allow for the hybridization to the first and the sequence portion of a treated nucleic acid, respectively, whereby the first and/or the second sequence portion of a treated nucleic acid and/or the at least one nucleotide between the first and/or the second sequence portion of a treated nucleic acid comprise at least one position which is to be analyzed for its methylation status.

A third sequence portion 7 is located between the first and the second sequence portion 5, 6 of the at least one oligonucleotide 4, which allows for the at least one oligonucleotide 4 to form a circle-like structure when the first and the second sequence portion 5, 6 of the at least one oligonucleotide 4 are hybridized to the first and the second sequence portion of the treated nucleic acid.

The third sequence portion 7 of the at least one oligonucleotide 4 shown here contains a first primer sequence 8 for binding of a first primer, a second primer sequence 9 for binding of a second primer. The first and second primer sequence 8, 9 are oriented such the first and the second primers have their 3' ends directed to each other when bound to the at least one oligonucleotide 4. This ensures that no amplification product can be generated form an at least one oligonucleotide 4 which has not been ligated to itself.

Furthermore, the third sequence portion 7 of the at least one oligonucleotide 4 shown here contains a first cleavage site 17 and a second cleavage site 18 for cleaving the at least one oligonucleotide 4. The first cleavage site 17 is located between the first and second primer sequence 8, 9, whereby the first primer sequence 8 is located 5' from the first cleavage site 17, and the second primer sequence 9 is located 3' from the first cleavage site 17. The second cleavage site 18 is located between the second sequence portion 6 and a tag sequence 19 for binding to an immobilized nucleic acid, whereby the tag sequence 19 is itself located at the 3' end of the second primer sequence 9.

In summary, the at least one oligonucleotide 4 shown in figure 3 A contains the following components from 5' to 3': a first sequence portion 5 for hybridizing to the treated nucleic acid, a third sequence portion 7, and a second sequence portion 6 for hybridizing to the treated nucleic acid, whereby the third sequence portion 7 contains from 5' to 3' a first primer sequence 8, a first cleavage site 17, a second primer sequence 9, a tag sequence 19, and a second cleavage site 18.

Two general embodiments of the method according to the present invention can be performed using an at least one oligonucleotide as shown in figure 3 A. According to the first general embodiment, the first and/or the second sequence portions 5, 6 of the at least one oligonucleotide 4 comprise at least one position that hybridizes to a position of the nucleic acid to be analyzed which contains either a methylated or unmethylated cytosine. In this embodiment, the first sequence portion 5 is positioned adjacent to the second sequence portion 6 without a gap between them when both the first and the second sequence portions 5, 6 are hybridized to the treated nucleic acid to be analyzed. Therefore, this embodiment can be used to measure the base conversion of methylated or unmethylated cytosine bases through a ligation reaction, which will only occur when no mismatches are present between the first and the second sequence portion 5, 6 of the at least one oligonucleotide and the nucleic acid to be analyzed. If bisulfite was used to treat the nucleic acid, then the conversion of unmethylated cytosine bases to uracil bases can be measured, ensuring complete conversion of all cytosine bases of the nucleic acid.

According to the second general embodiment, a gap of preferably one nucleotide is present between the first sequence portion 5 and the second sequence portion 6 when both the first and the second sequence portions 5, 6 are hybridized to the treated nucleic acid to be analyzed, thereby forming a circle-like structure. Here, an extension and a ligation reaction is used to analyze whether at the position of the gap a methylated or an unmethylated cytosine was present prior to the conversion treatment that makes the bases distinguishable.

To distinguish between a methylated and an unmethylated position, two extension reactions are performed in parallel, one with a first nucleotide that forms a base pair with the (treated) nucleotide representing the methylated form, and with a second nucleotide that forms a base pair with the (treated) nucleotide representing the unmethylated form that is present at the gap in the nucleic acid to be analyzed. If, for example, after a bisulfite treatment, a uracil is present at the site of the gap in the nucleic acid, then a dATP is introduced into the at least one oligonucleotide 4 and the ligation reaction can now occur, since both ends of the at least one oligonucleotide 4 are now in direct proximity to each other. Alternatively, if a methylated cytosine is present at the site of the gap in the nucleic acid, then a dGTP is introduced into the at least one oligonucleotide 4 prior to ligation.

The analysis or the nucleic acid is performed using at least two reactions in parallel, namely one with dATP (for an unmethylated position) and one with dGTP (for a methylated position). It is also possible to use dCTP and/or dTTP in further parallel reactions as control reactions, which will measure a background signal which can be used to correct the signals for the methylated and/or unmethylated positions. The signals obtained for the methylated and unmethylated positions can then be used to form a methylation coefficient for each CpG site.

The following steps of ligation, amplification and labeling are also performed for each reaction (methylated, unmethylated, control) separately, as described above. Only after the labeling step can the separate reactions be pooled, for example to analyze them on a microarray.

Following the ligation reaction, non-ligated at least one oligonucleotides are preferably removed using an exonuclease. To release the ligated circular at least one oligonucleotide 4 from the nucleic acid it hybridized to, the ligated circular at least one oligonucleotide 4 is cleaved using the first cleavage site 17, which can be a recognition site for a suitable restriction enzyme or the position of an at least one uracil base that is removable using uracil-N-glycosylase. The advantage of using at least one uracil base in the at least one oligonucleotide 4 in the first cleavage site 17 is that uracil-N-glycosylase will not only depurinate the at least one oligonucleotide 4, but also the bisulfite treated nucleic acid to be analyzed. Therefore, the heat treatment following uracil-N-glycosylase treatment will result in cleavage of the circular at least one oligonucleotide 4, and of the nucleic acid, so that the background signal in the amplification reaction is greatly reduced in one step.

Cleavage of the circular DNA molecule formed, i.e. the at least one oligonucleotide 4, at the first cleavage site 17 results in a rearrangement of the sequence portions of the at least one oligonucleotide 4, resulting in an at least one rearranged oligonucleotide 4'. The sequence portions of the at least one rearranged oligonucleotide 4' shown in figure 3 B are from 5' to 3' as follows: a second primer sequence 9, a tag sequence 19, a second cleavage site 18, a second sequence portion 6 for hybridizing to the treated nucleic acid, a first sequence portion 5 for hybridizing to the treated nucleic acid, and a first primer sequence 8. Since cleavage occurred somewhere within the first cleavage site 17, part of the cleaved first cleavage site 17 may still be present at at least one end of the at least one rearranged oligonucleotide 4'.

Due to this rearrangement, the orientation of the first and second primer sequence 8, 9 is now such that a first and a second primer can amplify the at least one rearranged oligonucleotide 4'. When the method according to the present invention is used to analyze multiple potential methylation sites, it is preferred that the first and the second primer sequences 8, 9 are unique for all at least one oligonucleotides 4 used for the multiple sites.

Following amplification, the amplification products are labeled with one dye for each reaction, that is with a first dye for the "methylated reaction", and a second dye for the "unmethylated reaction". A third and/or a fourth dye can be used for control reactions which might have been performed as described above.

The labeled amplification products can now be pooled and hybridized to nucleic acids immobilized on a microarray. In order to allow for the amplificates to hybridize to the immobilized nucleic acid molecules, the tag sequence 19 is exposed by performing a cleavage reaction using the second cleavage site 18 of the at least one rearranged oligonucleotide 4', which is preferably a recognition site for a restriction endonuclease.

The immobilized nucleic acids are arranged in a defined manner on the microarray, so that their sequence is correlated to their position on the microarray. The signals generated by the different dyes used are detected with suitable devices and the methylation status of each CpG site is determined using a suitable method as known in the art.

It is noted that each of the different sequence portions of the at least one oligonucleotide, namely the first, second and third sequence portion, the first and second primer sequence, as well as the first and second cleavage site and the tag sequence for binding to an immobilized nucleic acid and, if applicable, the zymogen sequence portion can at least partially overlap with at least one other sequence portion, if the particular sequences permit it.

### Figure 4:

A preferred use of the method according to the present invention is illustrated with respect to figure 4, which shows a flow diagram of the use of the present method.

A fresh-frozen paraffin-embedded tissue section, here a prostate cancer section, is histochemically analyzed using the method according to the present invention. General methods for performing histochemical analysis are known in the art (e.g. Larsson C et al. (2004) In situ genotyping individual DNA molecules by target-primed rolling-circle amplification of padlock probes. 1(3): 227-232).

First, bisulfite conversion is performed on the tissue section as described (see also Nuovo GJ (2004) Methylation-specific PCR in situ hybridization. Methods Mol Biol 287: 261-272). Shown on the left panel is a CpG position with a methylated cytosine, whereas the right panel shows an unmethylated cytosine that is converted into uracil with bisulfite. Upon completion of the conversion reaction, the genomic DNA is digested with a restriction endonuclease, followed by performing a 5' to 3' exonucleolysis. Then, an at least one oligonucleotide is added under conditions allowing for it to hybridize to the genomic nucleic acid. In the example shown, the at least one oligonucleotide contains a guanine base at the position to be analyzed. Therefore, ligation is only possible here when the at least one oligonucleotide is hybridized to the nucleic acid comprising a methylated cytosine base, whereas ligation is impossible with the uracil containing nucleic acid which was unmethylated prior to bisulfite treatment.

A detectable signal is generated using rolling circle amplification based fluorescence labeling. Analysis of the signals and overlay with a normal histological section shows which areas of the prostate contain a genomic acid with a methylated target sequence.

### EXAMPLE

### Detection of the methylation status of a single CpG of the human Septin 9 gene

Here, we describe a system for DNA methylation detection based on bisulfite-converted genomic DNA according to the present invention. This technology can be used to detect the methylation status of a single CpG on bisulfite treated DNA.

Specifically, the analysis of the methylation status of a single CpG site in the promotor region of the human Septin 9 gene (Ensembl Gene ID ENSG00000184640) is described. The methylation status of the investigated CpG is detected using a TaqMan™ real time polymerase chain reaction (PCR), that is known in the art.

### Materials and methods:

All sequences were written from left to right in a 5' to 3' orientation.

Sequence of the at least one oligonucleotide (probe) that is ligatable to itself if a particular methylation status is present in the human Septin9 gene before treatment with bisulfite:

CTAAAAACCATTATATAAACTTCCCCTACGGCTCAACGTTCCTATTCGGTTUUUT
TGCAAATGTTATCGAGGTCCGGCACGCACAGGTGGCGAATCTCTTTAAATCAAA
AACCCTACCCCAAAAC (SEQ. ID. No. 4)

Sequence of the human Septin9 gene before treatment with bisulfite (genomic sequence; the nucleotide that is to be analyzed regarding its methylation status is shown in bold):

GGAGTAAATACAGCAGGCGAAGGGGAAGCTCACACAATGGTCTCCAGCGCTCT
GGGGCAGGGCTTCTGAGGGGCGGGCCTGCCTCTGCCGGGACCTGGAGCCCC
CGCCCCTCGGAGAGGCTCCTAGGCTGACTTGGGCAGAG (SEQ. ID. No. 5)

Sequence of the human Septin9 gene after treatment with bisulfite (first and second sequence portion of the of the septin9 sequence, to which the first and the second sequence portion of the at least one oligonucleotide in form of a probe will hybridize to, are underlined; the nucleotide between the first and the second sequence portion, forming a gap, that is to be analyzed regarding its methylation status is shown in bold): 
GGAGTAAATATAGTAGGCGAAGGGGAAGTTTATATAATGGTTTTTAGCGTTTTGG
GGTAGGGTTTTTGAGGGGCGGGTTTGTTTTTGTCGGGATTTGGAGTTTTCGTTT
TTCGGAGAGGTTTTTAGGTTGATTTGGGTAGAG (SEQ. ID. NO. 6)
 Primer P1: CCGAATAGGAACGTTGAGCCGT (SEQ. ID. No. 7)
Primer P2: GCAAATGTTATCGAGGTCCGGC (SEQ. ID. No. 8)
TaqMan probe: Fam-ACGCACAGGTGGCGAATCTC-BHQ1 (SEQ. ID. No. 9)

The method according to the present invention is performed under the following conditions:

### 1. Bisulfite treatment:

Unmethylated cytosines are converted to uracils using a bisulfite method as previously described in (Berlin,K., Ballhause,M. and Cardon,K. (2005)). Use 1 µg of human genomic DNA isolated from human blood (Roche), and 1 µg of enzymatically methylated human male genomic DNA (Chemicon) DNA and make a bisulfite treament with each DNA in a separate reaction mixture. The reaction mixtures (50 µl) are subjected to bisulfite treatment (Berlin,K., Ballhause, M. and Cardon, K. (2005). After the bisulfite treatment, the DNA is recovered in a volume of 50 µl.

In this example, the cytosine of interest is located in the 5' region of the septin9 gene. The at least one oligonucleotide contains two hybridization sites that hybridizes to complementary sites in the bisulfite DNA and creates a circular conformation with a single-nucleotide gap between the termini of the probe.

### 2. Hybridization, gap-fill and Ligation:

Prepare three identical reactions, each containing 100 ng of bisulfite Roche DNA and furthermore, prepare three identical reactions containing 100 ng of bisulfite Chemicon DNA. Add to each reaction 12 amol of the probe (see above for sequence), 0.0625 units Ampligase (Epicenter) and 0.5 units Stoffel fragment DNA polymerase (Applied Biosystems) in 9 µl of 20 mmol/I Tris-HCL (pH 8.3), 25 mmol/I KCI, 10 mmol/l MgCl₂, 0.5 mmol/I NAD and 0.01 % Triton X-1 00.

To reaction tube 1, add 1 µl of dATP (Fermentas), to reaction tube 2, add 1 µl of dGTP (Fermentas) and to reaction tube 3, add 1 µl of dCTP (Fermentas). Do this for both types of bisulfite DNA and incubate each reaction mixture for 4 min at 20 °C, 5 min at 95 °C and 15 min at 58 °C and 1 min 37 °C.

In reactions in which the added nucleotide is complementary to the single-base gap, the DNA polymerase adds the appropriate nucleotide and a thermostable ligase closes the gap to form a covalently closed circular molecule that encircles the bisulfite strand to which it is hybridized.

### 3. Exonuclease treatment:

Thereafter, add 10 units Exonuclease I and 200 units Exonuclase III in 25 µl of 1.6 mmol/l MgCl₂, 10 mmol/l Tris-HCl (ph 8.3), 50 mmol/l KCl and incubate for 9 min at 37 °C and 20 min at 95 °C.

Exonucleases are added to digest linear probes in reactions where the added nucleotide was not complementary to the gap and excess linear probe in reactions where circular molecules were formed. The reactions are then heated to inactivate the exonucleases.

### 4. Uracil depurination and cleavage:

To release probes from bisulfite DNA, add 2 units of uracil-N-glycosylase (New England Biolabs) in 25 µl of 1.6 mmol/l MgCl₂, 10 mmol/l Tris-HCl (pH 8.3), 50 mmol/l KCl and incubate for 9 min at 37 °C. The uracil N-glycosylases depurinates the uracil residues in the probes. Advantageously, it also depurinates uracil residues present in the DNA that is to be analyzed, because it contains uracil residues due to bisulfite treatment. Heat the mixture for 20 min at 95 °C to release the probe from the template bisulfite treated DNA strand.

### 5. Amplification

For the amplification of the linearized probes containing the gap-filled nucleotide, incubate 2 units of AmpliTaq Gold (Applied Biosystems), 16 pmol of primer P1, and 16 pmol of primer P2 in 25 µl of 1.6 mmol/l MgCl₂, 10 mmol/I Tris-HCl (pH 8.3), 50 mmol/I KCI and 100 µmol/l dNTP. Use the following PCR program: 10 min at 95 °C (preactivation), than 28 cycles of 95 °C for 20 s, 65 °C for 45 s and 72 °C for 10 s.

### 6. Detection:

For the detection of the methylation status for the investigated cytosine with TaqMan real time PCR, use 10 µl of a 1:50 dilution of each PCR product and add 0.625 µmol/l of each primer P1 and P2, 0.2 µM of TaqMan Probe, 1 x Reaction buffer (Eurogentec), 3.0 mmol/l MgCl₂ (Eurogentec), 1 Unit HotGoldStarTaq polymerase (Eurogentec) and add water to a final volume of 20 µl.

Use the following real time PCR program on a ABI7900:
10 min at 95 °C (preactivation), than 45 cycles of 95 °C for 15 s, 65 °C for 30s, 72 °C for 10 s. Measure each sample in triplicates.

If the cytosine is methylated in the CpG of the Septin9 to be analyzed, amplification can only be detected in the reaction with a dGTP, because only in the presence of a dGTP will the extension reaction, and therefore the following ligation reaction be performed. Correspondingly, a signal in the reaction with the dATP will only be detected if the investigated cytosine is unmetylated. A possible unspecific background will be detected in the dCTP reaction.

The expected signal detection for the different DNA and different dNTP reactions is shown in table 1.

**Tab. 1:**

| | Roche DNA | Chemicon DNA |
|---|---|---|
| | | |
| dATP | + | - |
| dGTP | - | + |
| dCTP | - | - |

| | | |
|---|---|---|
| (+): amplification curve on the TaqMan ABI7900 (-): no amplification curve on the TaqMan ABI7900 | | |

The kind of results depicted in Tab. 1) would show that the cytosine of interest is methylated in Chemicon DNA und unmethylated in Roche DNA. The reactions with the added cytosine nucleotide will show a very low or no signal. This signal is a benchmark for unspecific reactions of the molecular inversion probe.

In the example described above, the gap between the first and the second sequence portion of the nucleic acid to be analyzed was one oligonucleotide wide. It is, however, also possible to chose a gap that is wider than one nucleotide. The length of the gap that can be filled is dependent on the particular sequence of the nucleic acid to be analyzed and can be up to ten nucleotides wide.

If, e.g. the nucleic acid to be analyzed has the sequence ACGGTCGCGATTCGGCG (SEQ. ID. No. 10), the gap could consist of the nucleotides GTCG. In this case, the methylation status of the C in the gap sequence GTCG can be analyzed with suited probes binding to the sequences on the 5' and 3' side of the sequence GTCG (underlined sequences). If the C to be analyzed was methylated and therefore not transformed by bisulfite, the gap is being filled in the extension step with CGTC. If, however, the C to be analyzed was unmethylated and therefore transformed to uracil (u) using bisulfite, the gap is being filled in the extension step with CAAC, as shown below.

methylated
ACGGTCGCGATTCGGCG
CAGC

unmethylated
ACGGTuGCGATTCGGCG
CaaC

## Claims

1. A method for the analysis of the methylation status of a nucleic acid, comprising the following steps:
• treating the nucleic acid with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases, and
• hybridizing to the treated nucleic acid an at least one oligonucleotide that is ligatable to itself if a particular methylation status is present in the nucleic acid, and
• ligating the at least one oligonucleotide to itself to form a circular DNA molecule if a particular methylation status is present in the nucleic acid, and
• amplifying the DNA molecule formed to detect the methylation status of the nucleic acid.

2. The method according to claim 1, wherein the nucleic acid to be analyzed contains a first sequence portion and a second sequence portion which are located adjacent to each other, and wherein the at least one oligonucleotide comprises:
- a first sequence portion for hybridizing to the first sequence portion of the treated nucleic acid,
- a second sequence portion for hybridizing to the second sequence portion of the treated nucleic acid, and
- a third sequence portion which is located between the first and the second sequence portion of the at least one oligonucleotide and which allows for the at least one oligonucleotide to form a circle-like structure when the first and the second sequence portion of the at least one oligonucleotide are hybridized to the first and the second sequence portion of the treated nucleic acid.

3. The method according to claim 2, wherein the first and/or the second sequence portion of the at least one oligonucleotide comprise at least one first base for the analysis of a methylated cytosine base which hybridizes to a treated methylated cytosine base and/or at least one second base for the analysis of an unmethylated cytosine base which hybridizes to a treated unmethylated cytosine base.

4. The method according to claim 2 or 3, wherein the at least one oligonucleotide forms a circle-like structure when the first and the second sequence portion of the at least one oligonucleotide are hybridized to the first and the second sequence portion of the treated nucleic acid.

5. The method according to any of claims 2 to 4, wherein the first sequence portion of the hybridized at least one oligonucleotide is ligated to the second sequence portion of the hybridized at least one oligonucleotide to form a circular DNA molecule.

6. The method according to any of claims 2 to 5, wherein the first and the second sequence portion of the treated nucleic acid are located adjacent to each other, with a gap of less than eleven nucleotides between them, and wherein the first sequence portion of the at least one oligonucleotide is reverse complementary to the first sequence portion of the treated nucleic acid, and wherein the second sequence portion of the at least one oligonucleotide is reverse complementary to the second sequence portion of the treated nucleic acid.

7. The method according to any of claims 2 to 6, wherein prior to ligating the first sequence portion of the hybridized at least one oligonucleotide to the second sequence portion of the hybridized at least one oligonucleotide, an extension reaction is performed with either
- a first deoxyribonucleotide, which forms a base pair with a treated methylated cytosine base, to form a circular DNA molecule if the nucleic acid to be analyzed was methylated at the position of the gap, or
- a second deoxyribonucleotide, which forms a base pair with a treated unmethylated cytosine base, to form a circular DNA molecule if the nucleic acid to be analyzed was unmethylated at the position of the gap,
wherein the first and the second deoxyribonucleotide are different from each other.

8. The method according to claim 7, wherein an additional extension reaction is performed with a third deoxyribonucleotide as a control, which does not form a base pair with neither a treated methylated cytosine base nor with a treated unmethylated cytosine base.

9. The method according to one of the preceding claims, wherein the nucleic acid is treated with a bisulfite containing solution, whereby unmethylated cytosine bases of the nucleic acid are converted into uracil bases, whereas methylated cytosine bases remain unchanged.

10. The method according to claim 7, wherein the first deoxyribonucleotide is dGTP, and the second deoxyribonucleotide is dATP.

11. The method according to claim 8, wherein the third deoxyribonucleotide is dCTP or dTTP.

12. The method according to any of the preceding claims, wherein the third sequence portion of the at least one oligonucleotide comprises
- a first primer sequence for binding of a first primer.

13. The method according to claim 12, wherein the third sequence portion of the at least one oligonucleotide further comprises
- a second primer sequence for binding of a second primer.

14. The method according to claim 12, wherein the first primer is used for initiating a rolling circle amplification.

15. The method according to claim 13, wherein the first primer and the second primer are used for initiating an exponential amplification.

16. The method according to claim 15, wherein the exponential amplification is a polymerase chain reaction, preferably a real-time polymerase chain reaction.

17. The method according to claims 12 to 16, wherein the first and the second primer sequences are oriented such that the DNA molecule formed is amplified only if the first sequence portion of the at least one oligonucleotide was ligated to the second sequence portion of the at least one oligonucleotide.

18. The method according to any of the preceding claims, wherein the third sequence portion of the at least one oligonucleotide comprises a tag sequence for hybridizing to an oligonucleotide that is immobilized on a substrate, preferably on a biochip.

19. The method according to claim 18, wherein the tag sequence is between 10 nucleotides to 30 nucleotides long, preferably between 15 nucleotides to 25 nucleotides long.

20. The method according to any of the preceding claims, wherein the third sequence portion of the at least one oligonucleotide further comprises
- a first cleavage site for cleaving the at least one oligonucleotide for releasing the at least one oligonucleotide from the treated nucleic acid.

21. The method according to claim 20, wherein the third sequence portion of the at least one oligonucleotide comprises at least one uracil base which is removable by uracil-N-glycosylase.

22. The method according to claim 21, wherein the at least one oligonucleotide is treated using uracil-N-glycosylase to remove the at least one uracil base and to create at least one abasic site.

23. The method according claim 22, wherein the at least one oligonucleotide is heated to cleave the at least one oligonucleotide at the abasic site to release it from the treated nucleic acid.

24. The method according to any of the preceding claims, wherein the third sequence portion of the at least one oligonucleotide further comprises
- a second cleavage site for cleaving the at least one oligonucleotide for making the tag sequence accessible so that the cleaved at least one oligonucleotide can hybridize to an oligonucleotide that is immobilized on a substrate.

25. The method according to any of the preceding claims, wherein at least one exonuclease is added after the ligation reaction to digest the at least one oligonucleotide that was not ligated.

26. The method according to any of the preceding claims, wherein the at least one oligonucleotide is cleaved using a restriction enzyme.

27. The method according to any of the preceding claims, wherein the amplified DNA molecule formed is labeled with either
- a first dye, if the extension reaction was performed with the first deoxyribonucleotide, or
- a second dye, if the extension reaction was performed with the second deoxyribonucleotide
wherein the first and the second dye can each generate a detectable and distinguishable signal.

28. The method according to claim 27, wherein the amplified DNA molecule formed is furthermore labeled with
- a third dye, if the extension reaction was performed with the third deoxyribonucleotide.

29. The method according to any of the preceding claims, wherein the signal generated from the first, second and/or third dye is detected.

30. The method according to any of the preceding claims, wherein the detected signal is quantitatively detected.

31. The method according to any of the preceding claims, wherein the detected signal stemming from the third dye is subtracted from the detected signal stemming from the first and/or second dye.

32. The method according to any of the preceding claims, wherein a multitude of the at least one oligonucleotide, which differ in their first and second sequence portions from each other, is used to analyze the nucleic acid at different sequence sites simultaneously.

33. The method according to claim 32, wherein the multitude of the at least one oligonucleotide comprises 2 to 2,000 different oligonucleotides, preferably 5 to 500, most preferably 50 to 200 different oligonucleotides.

34. The method according to claims 4 to 33, wherein the labeled amplified DNA molecule formed is hybridized with oligonucleotides immobilized on a microarray to detect the signal quantitatively.

35. The method according to claims 4 to 34, wherein a ratio is calculated of the signal stemming from a methylated and an unmethylated site.

36. The method according to any of the preceding claims, wherein the first sequence portion and/or the second sequence portion is between 5 nucleotides and 50 nucleotides in length, preferably between 10 nucleotides and 30 nucleotides in length, and most preferably between 15 nucleotides and 18 nucleotides in length.

37. The method according to any of the preceding claims, wherein the third sequence portion is between 15 and 50 nucleotides in length, preferably between 25 and 40 nucleotides in length, and most preferably between 30 nucleotides and 36 nucleotides in length.

38. The method according to any of the preceding claims, wherein the tag sequence is between 5 nucleotides and 50 nucleotides in length, preferably between 10 nucleotides and 40 nucleotides in length, and most preferably between 16 nucleotides and 19 nucleotides in length.

39. The method according to any of the preceding claims, wherein the ligation reaction is performed using a thermostable ligase.

40. The method according to claims 4 to 39, wherein the extension reaction is performed using a thermostable polymerase.

41. An oligonucleotide for analyzing the methylation status of a nucleic acid with a first and second sequence portion which are located adjacent to each other, which comprises
- a first sequence portion for hybridizing to the first sequence portion of the treated nucleic acid,
- a second sequence portion for hybridizing to the second sequence portion of the treated nucleic acid, and
- a third sequence portion which is located between the first and the second sequence portion of the at least one oligonucleotide and which allows for the at least one oligonucleotide to form a circle-like structure when the first and the second sequence portion of the at least one oligonucleotide are hybridized to the first and the second sequence portion of the treated nucleic acid,
**characterized in that** the first and the second sequence portion of the oligonucleotide only contain adenine, cytosine, and thymine, but no guanine.

42. A kit suitable for performing the method according to one or more of the claims 1 to 40, comprising the following components:
a) a chemical reagent or an enzyme for treating a nucleic acid, which alters the base pairing behavior of methylated cytosine bases and/or unmethylated cytosine bases of the nucleic acid such that methylated cytosine bases become distinguishable from unmethylated cytosine bases,
b) at least one oligonucleotide that is ligatable to itself, and
c) an enzymatic activity for ligating the at least one oligonucleotide and/or an enzymatic activity for amplifying a nucleic acid.

43. The kit according to claim 42, wherein the chemical reagent is bisulfite.

44. The kit according to claim 42 or 43, wherein the at least one oligonucleotide comprises
- a first sequence portion for hybridizing to a first sequence portion of a treated nucleic acid,
- a second sequence portion for hybridizing to a second sequence portion of the treated nucleic acid, and
- a third sequence portion which is located between the first and the second sequence portion and which allows for the at least one oligonucleotide to form a circle-like structure when hybridized to the treated nucleic acid.

45. The kit according to claim 42, 43 or 44, wherein the at least one oligonucleotide is the oligonucleotide according to claim 41.

46. Use of the method according to claims 1 to 40 or of an oligonucleotide according to claim 41 or of a kit according to claims 42 to 45 for diagnosis and/or prognosis of adverse events for patients or individuals, whereby these adverse events belong to at least one of the following categories:
undesired drug interactions; cancer diseases; CNS malfunctions; damage or disease; symptoms of aggression or behavioral disturbances; clinical;
psychological and social consequences of brain damages; psychotic disturbances and personality disorders; dementia and/or associated syndromes;
cardiovascular disease, malfunction or damage; malfunction, damage or disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and metabolic malfunction, damage or disease; headaches or sexual malfunction.

47. Use of the method according to claims 1 to 40 or of an oligonucleotide according to claim 41 or of a kit according to claims 42 to 45, for distinguishing cell types and/or tissues and/or for investigating cell differentiation.

48. Use of the method according to claims 1 to 40 or of an oligonucleotide according to claim 41 or of a kit according to claims 42 to 45, for identifying an indication-specific target in a nucleic acid, which is defined by a difference in the methylation of a nucleic acid derived from a diseased tissue in comparison to the methylation of a nucleic acid derived from a healthy tissue.

49. Use of the method according to claims 1 to 40 or of an oligonucleotide according to claim 41 or of a kit according to claims 42 to 45 for in situ diagnostics in a histological section.
